Europäisches Patentamt

European Patent Office

Office européen des brevets

(19)

(11) **EP 1 070 955 A2**

(12) **EUROPEAN PATENT APPLICATION**

(43) Date of publication:
   **24.01.2001 Bulletin 2001/04**

(51) Int Cl.$^7$: **G01N 21/35**, G01N 33/22

(21) Application number: **00306233.8**

(22) Date of filing: **21.07.2000**

(84) Designated Contracting States:
   **AT BE CH CY DE DK ES FI FR GB GR IE IT LI LU MC NL PT SE**
   Designated Extension States:
   **AL LT LV MK RO SI**

(30) Priority: **21.07.1999 GB 9917152**

(71) Applicant: **Zellweger Analytics Limited**
   **Nuffield Estate Poole, Dorset BH17 0RZ (GB)**

(72) Inventor: **Richman, Lee**
   **Sandford, Wareham Dorset BH20 7BX (GB)**

(74) Representative:
   **Hedley, Nicholas James Matthew et al**
   **Stephenson Harwood**
   **One, St. Paul's Churchyard**
   **London EC4M 8SH (GB)**

(54) **Measurement of gas calorific value with infrared radiation**

(57)   A method of measuring the calorific value of gas in a gaseous space using a two channel non-dispersive infrared detector in circumstances in which the gas comprises at least one flammable gas from a group of gases ("target gases") but the composition of the gas in the space may be unknown, the method comprising:

   a) transmitting infra-red radiation through the gas in the space(14);
   b) passing the infrared radiation through a single static filter (18) that transmits radiation only in one or more sample wavelength hands, each of the target gases absorbing radiation in the sample wavelength hand(s);
   c) measuring the aggregate intensity of radiation that has passed through the space in the sample wavelength band ("sample intensity") by means of a sensor (22);
   d) passing the infrared radiation through a single static filter (20) that transmits radiation only in one or more reference wavelength bands, each of the target gases absorbing radiation to a lesser extent in the reference wavelength hands than in the sample wavelength bands;
   e) measuring the aggregate intensity of radiation that has passed through the space in the reference wavelength bands ("reference intensity") using a sensor (24); and
   f) calculating the ratio between the sample intensity of the gas in the gaseous space to the reference intensity of the gas in the gaseous space,

   The number of sample and reference wavelength bands is at least three. The sample and the reference wavelength bands are so selected that, for each target gas, the calorific value of that gas is approximately equal to a fixed function ($F_k$) of the ratio of (a) the sample intensity for that target gas alone to (b) the reference intensity for that target gas alone, the fixed function ($F_k$) being the same for all the target gases. The calorific value of the gas in the gaseous space can be derived (irrespective of the exact composition of target gases in the gas space) by applying the fixed function ($F_k$) to the ratio calculated in step f). The method allows the calorific value of a flammable gas, e.g. natural gas, to be calculated within limits irrespective of the composition of the gas.

Fig.1.

**Description**

## INDUSTRIAL FIELD

The present invention relates to the use of infrared detection to measure the calorific value of gases (which term includes vapours), especially hydrocarbon gases.

## BACKGROUND ART

The use of non-dispersive infrared spectroscopy to detect gases and to measure their properties is well established. It essentially involves transmitting infrared radiation along a path in a space being monitored; the wavelength of the infrared radiation is chosen so that it is absorbed by the gas of interest (hereafter called the "target gas") but not substantially absorbed by other gases in the space. The level of absorption should provide a measure of the amount of target gas in the space.

However, factors other than absorption by the target gas also attenuate the infrared radiation. Therefore infrared radiation at a wavelength at which the target gas does not exhibit substantial absorption is also transmitted through the space being monitored to act as a reference. The radiation at the reference wavelength will ideally be attenuated by the extraneous factors to approximately the same degree as is the radiation that is absorbed by the target gas. The effects of the extraneous factors can then he reduced or even eliminated by taking the ratio between the signal obtained at the wavelength where the target gas does absorb (the "sample wavelength") and the signal obtained at the wavelength where the target gas does not significantly absorb (the "reference wavelength") to derive a measure of the amount of target gas in the space.

All the $C_{1-7}$ hydrocarbons are gaseous or volatile; the $C_{1-4}$ alkanes are gaseous and found in natural gas. It is not feasible to provide separate systems for detecting each hydrocarbon and therefore it is necessary that a single system should be capable of detecting all these hydrocarbons of interest. However, the hydrocarbons all have different spectra and so the selection of a single sample wavelength and a single reference wavelength that can achieve uniform responsivity for all of the $C_{1-7}$ hydrocarbons (or $C_{1-4}$ alkanes) is not possible.

However, all gaseous hydrocarbons will absorb radiation to varying extents at wavelengths in the 2.3 to 2.4 μm and 3.3 to 3.4 μm regions, which can be used as the sample wavelength.

The response of such conventional infrared detectors can vary by as much as 300% between different hydrocarbon gases, which makes an accurate measurement by infrared detectors of the amount of gas present impossible unless the composition of the gas is known.

It is possible to use infrared gas detectors to measure the amount of a flammable gas of known composition in a space and so provide a measure of its calorifie value. However, this is difficult in the case of natural gas, the constituents of which vary substantially and which can even include inert (i.e. non-flammable) gases, e.g. natural gas can consist of 85 to 99% methane, 0 to 10% ethane, 0 to 3% propane, 0 to 1% butane, 0 to 10% nitrogen and 0 to 2% carbon dioxide. As mentioned above, the response of typical infrared detectors between different hydrocarbons can vary by as much as 300%, which generally makes them unsuitable for measuring calorific values of natural gas and other parameters associated with hydrocarbon gases of unknown composition.

The measurement of the calorific values of natural gas mixtures is therefore generally performed by complex, expensive equipment, e.g. gas chromatographs and flame ionisation detectors, but such equipment is prone to drift. For this reason, the calorific value of natural gas is only measured at a limited number of points. It would be desirable to have a simple, cheap and stable method of measuring calorific values of mixtures of hydrocarbon gas.

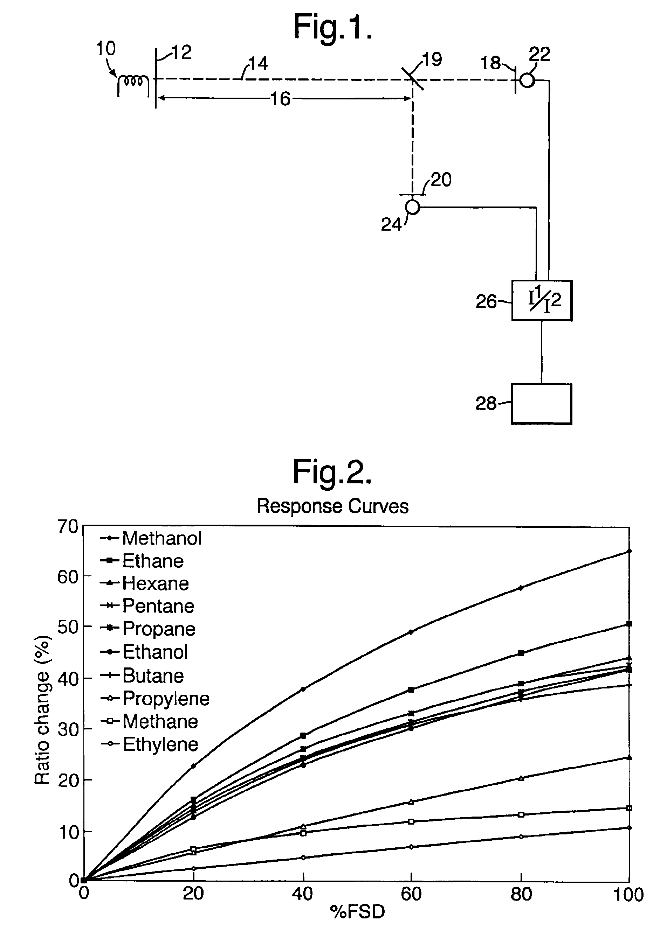

Figure 2 shows the response of a conventional sensor to various hydrocarbon and alcohol gases/vapours. The X-axis represents the percentage of the gas/vapour concerned in the atmosphere. The Y-axis is the ratio change (expressed as a percentage) in the Gas Measurement Ratio (GMR) as compared to the GMR value when there is no target gas in the atmosphere. The (iMR is the ratio between (a) the signal at the sample wavelength band(s) and (b) the signal at reference wavelength band(s). The ratio change is calculated using the following formula:

$$\text{Ratio Change} = 100 * \{1 - \frac{\text{GMR in the presence of the target gas}}{\text{GMR with no target gas}}\}$$

To get a uniform response from all hydrocarbons it is desirable that, in the range of interest, all the curves are parallel, which is clearly not the case in Figure 2.

It has been proposed (see for example GB-1,402,301, GB-1,402,302, US-4,567,366, EP-0,744,614 and GB-2,163,251) to use two reference wavelengths located on either side of a sample wavelength and to take, as the reference signal, the average of the signals at the two reference wavelengths.

PCT Application GB98/02991 describes an infrared detector that, through the selection of sample and reference wavelength bands, allows the detection of $C_{1-7}$ hydrocarbons even in fog. The sample wavelength band is at 2300nm and has a full width half maximum (FWHM) of 50 nm. The reference wavelengths have a first band centred around 2215 nm with a FWHM of 25 nm and a second band having a central wavelength of 2385 nm and a FWHM of 25nm. Because the sample wavelength band lies midway between the two reference wavelength bands, the detector provides good detection of $C_{1-7}$ hydrocarbons even in the presence of rain and fog as compared to conventional detectors.

CA-A-2 236 865 describes a method of measuring the calorific value of a gas by transmitting radiation through a gas in three distinct sample wavelengths and one reference wavelengths. The signals at the different wavelengths are modulated at different frequencies before being transmitted through the gas and the signal that has passed through the gas is then demodulated to derive the transmittance of the gas at each of the wavelengths. The calorific value is calculated from the ratio between (a) the weighted aggregate of the transmittance at the sample wavelengths and (b) the transmittance at the reference wavelengths. Such an arrangement of modulating different signals is complex and expensive to implement.

GB-A-2 228 568 describes an apparatus for discriminating between different hydrocarbon gases The principal objective of this apparatus is discrimination between pipeline gas and non-pipeline gas to assist tracking of the source of a build-up of flammable gas. Separate measurements at three distinct wavelengths are made. By comparing the IR absorption of gas vapour at different sample wavelengths, it is possible to determine the composition of the gas. However, the apparatus is complex and cannot measure calorific value

GB-A-2 163 251 describes a methane sensor using a rotating interference filter. By rotating the interference filter, it is possible to change the centre wavelength of the filter and scan the transmitted wavelength over a range of about 60nm to facilitate accurate discrimination and determination of methane gas concentration.

It is known to use multiple separate wavelength measurements to achieve uniform responsivity of an infrared detector (a) by bringing separate filters for each wavelength band successively into the beam of detecting light, e.g. by mounting the filters on a wheel that rotates in the beam of detecting light or (b) by using three or more channels, i. e. three or more separate beams and detectors for each band or (c) by modulating the light in the separate hands differently and demodulating the signal from the detector to discern the intensity of radiation in each band. Each arrangement has severe disadvantages in practice, particularly when exposed to harsh weather conditions. The use spinning wheels in (a) is prone to disruption when used outside a controlled environment. Likewise the use of more than two beams and detectors in (b) increases the cost of an instrument and requires the alignment of the beams. The modulation of the radiation in (c) substantially increases the cost and complexity of the instrument.

It is known that two channel non-dispersive infrared (NDIR) detectors are robust. Two channel non-dispersive infrared detectors have two fixed filters, one located in the sample channel in front of a sensor and the other located in the reference channel in front of another sensor and so there is no need to provide a mechanical arrangement for bringing filters into alignment with a sensor. Furthermore the use of only two channels makes alignment of the radiation source simpler than if more than two channels are provided.

It is an object of the present invention to provide a method of measuring the calorific value of a mixture of gases ("target gases") using a two channel non-dispersive infrared detector having fixed filters when the target gases each have different calorific values and are present in the mixture in unknown proportions.

## DISCLOSURE OF THE INVENTION

According to the present invention, there is provided a method, as set out in the accompanying claims, of measuring the calorific value of a gas in a gaseous space containing at least one flammable gas from a group of gases ("target gases") in circumstances in which the composition of the gas in the space may be unknown, There is also provided a detector for measuring the calorific value of a gas and a method of selecting filters for use in such a method and detector, as set out in the accompanying claims

Thus, according the present invention, the sample and reference wavelength bands are specifically tailored such that, for each target gas, the calorific value for that target gas (CV) is approximately equal to a constant function $F_k [\ ]$ of the ratio of (a) the measured aggregate intensity of radiation that has passed through that target gases at the sample wavelength band(s) ($\Sigma I(S)$) divided by the corresponding aggregate intensity of that target gas at the reference wavelength band(s) ($\Sigma I(R)$), i.e.

$$CV = F_k \left\lceil \frac{\Sigma I(S)}{\Sigma I(R)} \right\rceil$$

for each of the target gases.

The function $F_k$ can be derived from Beer's law and, in theory, is a natural logarithm function of $\Sigma(S)/\Sigma I(R)$ multiplied by a constant. However, due to non-linearities and varying molar absorption coefficients across the wavelength bands, this is only an approximation and usually the exact function will be derived empirically for the detector used to obtain the best fit for the data obtained.

If the sample and reference wavelengths are tailored in this way, the calorific value (CV) of a mixture of gases can be derived by applying the function $F_k$ to the ratio:

$$\frac{\text{the signal at the sample wavelength band(s) for a mixture of gases}}{\text{the signal at the reference wavelength band(s) for the mixture of gases}}$$

to give an approximate measure of the calorific value of the gas mixture irrespective of the exact composition of the gas in the gaseous space.

Thus, the present invention tailors the sample and reference wavelength bands (and other characteristics of the filter) used in an infrared gas monitor so that it is possible to derive from the ratio of the signal of the gas mixture at the sample to that at the reference wavelengths to provide a signal giving a real time direct measure of the calorific value of the mixture irrespective of the composition of mixture (within limits). The tailoring of the reference wavelength bands is achieved by suitably choosing (a) the wavelength(s) of the reference bands, (b) the bandwidth of the reference bands and (c) the absorption by the filter of each band. It is therefore probable that the reference bands will be asymmetrically arranged with respect to the sample band(s) and the reference bands will have different bandwidths.

Approximate values of the sample and reference wavelength bands can be derived from theoretical considerations, as described above. The bandwidths are then altered empirically using the following technique: if, when the function $F_k$ is applied to the ratio of the sample signal to the reference signal for a given target gas (gas 1), a higher calorific value is generated than is the case but the correct calorific value is generated for another target gas (gas 2), then the reference wavelength bands can be adjusted to increase the absorption of gas 1 at reference wavelengths where gas 1 has strong absorption as compared to gas 2 and thereby decrease the ratiometric response for target gas 1 and hence decrease the value of the calorific value generated in respect of that target gas. By a judicious tailoring of the reference wavelength bands for the target gases of interest, it is possible to achieve an approximately uniform output of calorific value across a range of target gases.

A single infrared source beam that contains both the sample and the reference wavelength bands may be transmitted through the gas mixture or separate beams may be provided. Indeed, the beam can include a broad spectrum of wavelengths. including the sample and reference wavelength bands. A filter may be inserted in the infrared beam prior to entering the gas space being monitored to limit to the sample and reference wavelengths only the radiation being transmitted through the gas space, but such a filter is not necessary if the sample and reference filters are provided immediately in front of respective sensors in the detector.

When more than one band is provided for either the sample or the reference wavelengths, a single multi-bandpass filter should be used since the alternatives (the use of separate beams or a mechanical arrangement to bring separate filters in turn into a beam) give rise to significant operational performance and reliability problems and add to the cost. Furthermore, the use of an additional reference beam makes the system expensive and prone to obscuration problems.

The present invention provides a simple and low-cost solution to the monitoring of calorific values of gases. No attempt is made to determine what the constituents of the gas are since that adds to the complexity and cost of implementation. The present invention can determine the calorific value of a gas mixture without knowing or attempting to know the constituents of the mixture.

## DETAILED DESCRIPTION OF DRAWINGS

The present invention will be further described by way of example only with reference to the following drawings in which:

Figure 1 is a schematic view of the detector of the type used in the present invention and in the prior art. The difference between the present invention and the prior art lies in the nature of the filters, as explained in further detail below;

Figure 2 is a graph showing the change in the signal from a conventional, point non-dispersive infrared detector with changing concentrations of a range of common hydrocarbon and alcohol gases and vapours:

Figure 3 is a graph showing the transmittance of the sample and reference filters of a detector according to the present invention;

Figure 4 is a graph similar to Figure 2 showing the change in the signal from an infrared detector according to the present invention with changing concentrations of a range of common hydrocarbon and alcohol gases and vapours; and

Figure 5 is a graph showing the change in the signal from an infrared detector according to the present invention against calorific value for changing concentrations of a range of hydrocarbon found in natural gas.

## DETAILED DISCLOSURE OF PREFERRED EMBODIMENTS

Referring initially to Figure 1, there is shown an infrared detector for use in accordance with the present invention. It has a source of infrared radiation, which in the present invention is preferably a lamp 10, placed behind a filter 12 that transmits infrared radiation in sample and reference wavelength bands, as described in further detail below. The beam 14 transmitted by filter 12 passes along beam path 16, which can vary in length from a few centimetres upwards, and is attenuated by material in that path. The beam 14 attenuated in this way is incident on a receiver 17 containing a beam splitter 19; filters 18 and 20 and corresponding intensity measuring sensors 22 and 24 are placed in the two parts of the split beams. Filter 18 transmits radiation only at the sample wavelengths and filter 20 transmits radiation only at the reference wavelengths.

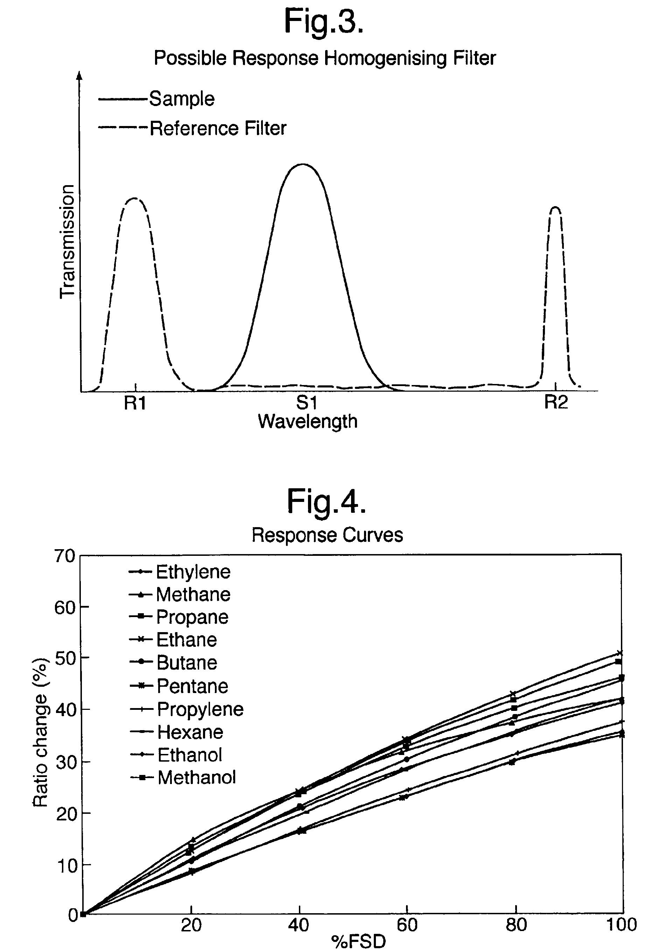

Filter 18 is a single bandpass filter transmitting radiation in one distinct sample wavelength band and filter 20 is a dual bandpass filter transmitting radiation in two distinct reference infrared wavelength bands, which preferably lie either side of the sample band of filter 18, as shown in Figure 3, where the reference wavelength bands are shown in broken lines and the single sample wavelength band is shown by the continuous line.

The signals from sensors 22 and 24 are fed into a circuit 26 that can calculate the ratio between these two signals and apply the function $F_k$ to provide an output to display 28 of the calorific value of the gas in the space traversed by the beam 14. If there is target gas in the path of the beam 14, radiation in the sample wavelength band is absorbed by the target gas whereas radiation in the reference wavelength bands is not (or at least not to the same extent). The ratio of the intensity signal of sensor 22 (measuring the sample wavelengths) as compared to the signal of sensor 24 (measuring the reference wavelengths) will therefore fall in the presence of a target gas.

The essential feature of the present invention is the tailoring of the sample and reference wavelengths to give a uniform response for the calorific value to be measured for a range of gases, which may be a mixture or it may be that an individual gas is present alone. The process of designing and tuning the response characteristics is preferably as follows:

1. Selecting one or more sample wavelengths where all the target gases absorb radiation, ideally where all the target gases absorb strongly and with similar strengths.

2. Selecting reference wavelengths; generally, it will be necessary to choose two or more reference wavelengths, at least one of which is strongly absorbed by some of the target gases and one of which is weakly absorbed by some or most of the target gases. It may well be that in one of the reference bands, a given target gas will absorb more strongly than in the sample wavelength hand! However, if that is the case, the summation of the reference absorption taken across the all the reference hands for that gas will be less than that in the sample hand(s). The reference wavelengths must be realisable on a single interference filter. Also, preferably, the reference wavelengths are located either side of the sample wavelength band(s) to minimise the attenuating effect of dirt. However for best uniformity of response, the reference bands will generally not be symmetrically distributed about the sample wavelength band(s) when implementing the present invention.

3. Calculating the intensity of radiation transmitted through each of the target gases at the sample wavelengths and then calculating the relationship between the intensity and the calorific value for each of the target gases. Then, the relationships between the calorific value and the absorption of all the target gases are compared with each other, preferably by calculating and expressing the said relationship of each gas relative to the other gases as a ratio;

4. Optimising the reference wavelengths to decrease the reference intensity (i.e. increase the absorption of the target gas at reference wavelengths) for target gases. which, on the basis of their ratiometric response, appear to provide too high a reading of calorific value (the term "ratiometric response" is the response obtained by dividing the signal from the sample wavelength band(s) by the signal from the reference wavelength band(s)).

5. Optimising the reference wavelengths to increase the reference intensity (i.e. decrease the absorption of the target gas at reference wavelengths) liar target gases, which, on the basis of their ratiometric response, appear to provide too low a reading of calorific value

6. Fine-tuning of the response by introducing deliberate imbalances between the relative bandwidths of the reference bands. This enables further, more precise control of the fractional effect of reference absorption on the overall reference signal.

Applying the above design process, it is possible to arrive at a spectrum for filters typically as shown in Figure 3, where the sample filter transmittance against wavelength is shown by a solid line and the reference filter transmittance against wavelength is shown by a broken line. It will be immediately apparent that the bandwidths of the two reference wavelength bands are not equal and neither is their strength. This is because. in order to achieve a uniform signal from each of the target gases giving a measure of the calorific value that is substantially independent of the composition of the target gas, the reference wavelength bands have been tailored as described above.

Naturally, the precise transmittance characteristics of the reference filter will depend partly on the sample wavelengths chosen, partly on the relationship between the absorption at sample wavelengths and the calorific values of the gases and partly on the nature of the absorption spectra at the reference wavelengths for each target gas.

Figure 4 shows the change in the signal in a monitor using filters of the present invention and it can be seen that the response is substantially more uniform than the corresponding arrangement using conventional filters shown in Figure 2.

## EXAMPLE

The present invention can determine the calorific value of natural gas at various points in a gas distribution network. Currently, this is performed by complex, expensive, drift-prone equipment such as gas chromatographs and flame ionisation detectors. Because the equipment is expensive and needs close supervision, the number of points where the use of such equipment is justified is relatively small. It would be desirable to provide simple, low cost, stable equipment for determining the calorific value of natural gas. This is reinforced by the fact that several suppliers can supply natural gas to the national natural gas distribution network in the UK. Since the calorific value of natural gas can vary by as much as 25%, it is important to know the calorific value of the gas supplied by each supplier.

The principal constituents of natural gas are methane (85-99%), ethane (0-10%), propane (0-3%), butane (0-1%), nitrogen (0-10%) and carbon dioxide (0-2%). The calorific value of different types of natural gas varies from about 9.86 to 12.17 kWh/cubic meter. depending on the concentrations of these constituents. An ideal device for measuring the calorific value of natural gas would produce an output that is proportional to the calorific value, irrespective of the precise constitution of the measured gas.

It is convenient to make certain assumptions in order to calculate suitable sample and reference wavelengths to produce an infrared detector that will directly measure calorific value across a range of gases so as to simplify the calculation, at least in the first instance. In the present case, the following assumptions were made:

1. Infrared absorption of the sample and reference wavelengths is only exhibited by alkanes present in the natural gas i.e. nitrogen and carbon dioxide do not appreciably absorb infrared radiation at the sample and reference wavelengths.
2. The relative abundances of the alkanes in natural gas only vary within the ranges given above; this significantly reduces the range of gas mixtures for which the device needs to be able to produce an accurate calorific value.
3. The absorption properties of each gas can be modelled by Beer's Law with a single absorption coefficient for each filter centre wavelength.
4. The net effect of absorption by the gas mixture can be modelled by multiplying the transmittances calculated for each of the individual gas components.
5. The properties of the optical filters can be represented by a simple model. including, centre wavelength, mean transmittance and bandwidth (full width half maximum (FWHM)).

Applying these assumptions, a gas measurement ratio (GMR) can be estimated by applying the formula derived from Beers Law set out below. The GMR will be the signal derived from dividing the radiation intensity measured at a single sample wavelength band (S) (i.e. by sensor 22) by the summed radiation intensity measured at two reference wavelength bands (RI and R2) (i.e. by sensor 24):

$$GMR = \frac{I(S) \cdot T_F(S) \cdot W_F(S) \cdot e^{-((\alpha 1(S).[K1] + \alpha 2(S).[K2] + \alpha 3(S).[K3] + \alpha 4(S).[K4])L)}}{[I(R1) \cdot T_F(R1) \cdot W_F(R1) \cdot e^{-((\alpha 1(R1).[K1] + \alpha 2(R1).[K2] + \alpha 3(R1).[K3] + \alpha 4(R1).[K4])L)} + \dots I(R2) \cdot T_F(R2) \cdot W_F(R2) \cdot e^{-((\alpha 1(R2).[K1] + \alpha 2(R2).[K2] + \alpha 3(R2).[K3] + \alpha 4(R2).[K4]L)}]}$$

where:

I(S) = Intensity at sample wavelength
I(R1) = Intensity at reference wavelength (1)
I(R2) = Intensity at reference wavelength (2)
$T_F$ (S) = Transmission of sample filter
$T_F$ (R1) = Transmission of reference filter at wavelength (R1)
$T_F$ (R2) = Transmission of reference filter at wavelength (R2)
$W_F$ (S) = Effective bandwidth of sample filter
$W_F$ (R1) = Effective bandwidth of reference filter at wavelength (R1)
$W_F$ (R2) = Effective bandwidth of reference filter at wavelength (R2)
$\alpha 1(X)$ = Absorption coefficient of methane at wavelength (X)
$\alpha 2(X)$ = Absorption coefficient of ethane at wavelength (X)
$\alpha 3(X)$ = Absorption coefficient of propane at wavelength (X)
$\alpha 4(X)$ = Absorption coefficient of butane at wavelength (X)
[K1] = Concentration of methane
[K2] = Concentration of ethane
[K3] = Concentration of propane
[K4] = Concentration of butane
L = Path length of measurement cell

Using the above formula and the absorption spectra of the various gases, the response to different types and concentrations of natural gas can be estimated. This enables a set of ratiometric response curves to be produced for a particular combination of sample and reference filters. The objective is to optimise the filter pair design such that, over the range of interest, the ratiometric response of different natural gases follows the same curve when plotted against calorific value. Due to non-linearities and non-Beer's Law. behaviour, it would only possible to achieve the required uniformity of response over a relatively limited range. When performing filter optimisation, it is therefore important to restrict the model gas variations to a realistic minimum.

The simplifying assumptions given above enable a device's theoretical gas response to be modelled relatively easily, using the absorption spectra of the gases concerned. However, in order to achieve an exact uniformity of calorific value response, it would be necessary to take account of all effects that contribute to the device's overall response. This would require the model to include the following:

1. Accurate, high resolution, absorption spectra for each of the gases found in the natural gas.
2. Correction for interaction between the natural gas constituents which effect their absorption spectra.
3. Compensation for gas pressure and temperature effects.
4. Accurate transmittance characteristics for real (non-idealised) infrared interference filters (including effects due to illumination geometry).
5. A realistic model of the infrared source characteristics.
6. Spectral responsivity of the infrared detectors.
7. Transmission, reflection and refraction characteristics of any optical components used, including beamsplitters, windows, mirrors and lenses.

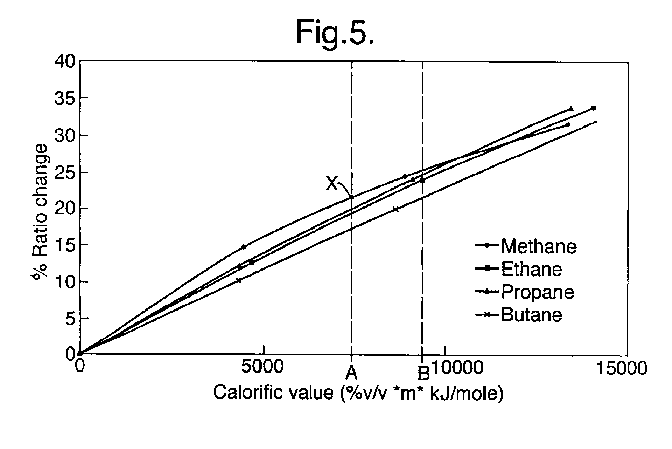

The quantity and quality of data required, combined with the considerable computational complexity can make such a model impractical but such factors can be allowed for by making empirical changes in the function used to calculate the calorific value from the ratiometric response. In order to exploit this technique in the absence of a suitably precise model, experimental measurements were performed on designs believed to be close to meeting the requirement. The best results obtained thus far are shown in Figure 5, which will now be discussed.

The required operating range of calorific value is between limits A and B shown in Figure 5. Over this range, the absolute and relative responses of the four main alkanes found in natural gas are very similar. The minimum percentage of methane in natural gas is 83% (v/v). which corresponds to point X on the methane curve The response curve to any particular natural gas mixture will therefore always be greater than point A, which is the calorific value of a gas containing 83% (v/v) methane. Other flammable natural gases constituents will add to the calorific value of the natural gas and point B corresponds to the highest expected calorific value of natural gas. When optimising the design, it is the relative responsivity to the different alkanes starting from point X that is important (i.e. the slope of the various curves). From Figure 5, the relative responsivities of the four alkanes (as compared to the responsivity of methane) from point X are:

| | |
|---|---|
| Methane | 1 : 1 |
| Ethane | 1 : 1.35 |
| Propane | 1 : 1.35 |
| Butane | 1 : 1.29. |

Therefore, a reduction in the relative responsivity to ethane and propane of 1/1.35 (i.e. -26%) will make the largest improvement in overall calorific response uniformity. However, this will also result in a reduction of 26% in the butane contribution (which should ideally be reduced by only 22.5%) but since the amount of butane in natural gases is relatively small compared to ethane and propane, this under-reading will he relatively unimportant.

The required reduction in ethane, propane and butane responsivity can he achieved by modifying the R2 passband in the equipment described in PCT Application GB98/02991 to a centre wavelength of 2.425 pm and a FWHM of 30nm. With these modifications it will be possible to measure natural gas calorific values to within $\pm 1\%$.

The specifications for the filters required to produce this natural gas calorific value measuring device are as set out below:

| **Sample Filter** | |
|---|---|
| Centre wavelength | 2300nm $\pm$ 5nm |
| FWIIM | 50nm $\pm$ 5nm |
| Peak Transmission | >50% |

| **Reference Filter** | |
|---|---|
| Centre wavelengths | R1 = 2165nm $\pm$ 5nm |
| | R2 = 2425nm $\pm$ 5nm |
| FWHM | R1 = 25nm $\pm$ 5nm |
| | R2 = ((Actual FWHM of R1)*(1.2 $\pm$ 0.1)) |
| Peak Transmission | >50% |
| Transmission Ratio (T(R1)/T(R2)) | >0.95:1 & < 1.05:1 |

Although the present invention has been described in connection with the measurement of the calorific value of natural gas, it is not limited to that application and can be applied to the measurement of the calorific values of other possible mixtures of flammable gases.

## Claims

1. A method of measuring the calorific value of gas in a gaseous space using a two channel non-dispersive infrared detector in circumstances in which the gas comprises at least one flammable gas from a group of flammable gases ("target gases") but the composition of the gas in the space may be unknown, the method comprising:

> a) transmitting infra-red radiation through the gas in the space;
> b) before or after step a) passing the infrared radiation through a single static filter that transmits radiation only in one or more sample wavelength bands, each of the target gases absorbing radiation in the at least one sample wavelength band;
> c) measuring the aggregate intensity of radiation that has passed through the space in the one or more sample wavelength band ("sample intensity");
> d) before or after step a), passing the infrared radiation through a single static filter that transmits radiation only in one or more reference wavelength bands, each of the target gases absorbing radiation to a lesser extent in the one or more reference wavelength hands than in the one or more sample wavelength bands;
> e) measuring the aggregate intensity of radiation that has passed through the space in the one or more reference wavelength bands ("reference intensity"); and
> f) calculating the ratio of the sample intensity of the gas in the gaseous space to the reference intensity of the gas in the gaseous space,

wherein the number of said sample and reference wavelength hands is at least three and the sample and reference wavelength bands are so selected that, for each target gas, the calorific value of that gas is approximately equal to a fixed function ($F_k$) of the ratio of (a) the sample intensity for that target gas alone to (b) the reference intensity for that target gas alone, the fixed function ($F_k$) being the same for all the target gases and wherein the method comprises a further step of:

> g) applying the said fixed function ($F_k$) to the ratio calculated in step f) to derive the calorific value of the gas in the space irrespective of its composition of target gases.

2. A method as claimed in claim 1, wherein a single sample wavelength band is used.

3. A method as claimed in claim 1 or claim 2, wherein at least two reference wavelength bands are used, which are preferably located on either side of the sample wavelengths in the spectrum..

4. A method as claimed in claim 3, wherein at least one of the reference wavelength bands is strongly absorbed by at least some of the target gases and at least one of the reference wavelength bands is weakly absorbed by at least some (and preferably all) of the target gases.

5. A method as claimed in claim 3 or 4, wherein the bandwidths of the reference wavelength bands are different.

6. A method as claimed in any one of claims 1 to 5, wherein the fixed function is a natural logarithm function.

7. A two channel non-dispersive infrared detector for measuring the calorific value of a gas in a gaseous space in circumstances in which at least one gas from a group of gases ("target gases") may be present in the space hut the composition of the gas in the space may be unknown, the apparatus comprising:

> a) a radiation source for transmitting infrared radiation through the gas;
> b) a single static filter that transmits radiation only in one or more sample wavelength bands, each of the target gases absorbing radiation in the at least one sample wavelength bands;
> c) a sensor for measuring the aggregate intensity of radiation that has passed through the space in one or more sample wavelength band(s) ("sample intensity");
> d) a single static filter that transmits radiation only in one or more reference wavelength bands, each of the target gases absorbing radiation to a lesser extent in the one or more reference wavelength hands than in the one or more sample wavelength bands
> e) a sensor for measuring the aggregate intensity of radiation that has passed through the space in the one or more reference wavelength bands ("reference intensity");

wherein the number of said sample and reference wavelength bands is at least three and the sample and reference wavelength bands are so selected that, for each target gas, the calorific value of that gas is approximately equal to a fixed function ($F_k$) of the ratio of (a) the sample intensity for that target gas alone to (b) the reference intensity for that target gas alone, the fixed function ($F_k$) being the same for all the target gases and wherein the detector further includes:

> f) a processor for calculating the ratio of the sample intensity of the gas in the gaseous space to the reference intensity of the gas in the gaseous space and for applying the said fixed function ($F_k$) to the ratio calculated to derive the calorific value of the gas in the space irrespective of its composition of target gases.

8.  An apparatus as claimed in claim 7, wherein a single sample wavelength band is used.

9.  An apparatus as claimed in claim 7 or claim 8, wherein two or more reference wavelength bands are used and a single filter transmits the reference wavelength bands to the sensor, which are preferably located on either side of the sample wavelengths in the spectrum.

10. An apparatus as claimed in claim 9, wherein at least one of the reference wavelength bands is strongly absorbed by at least some of the target gases and at least one of the reference wavelength bands is weakly absorbed by at least some (and preferably all) of the target gases.

11. An apparatus as claimed in claims 9 or claim 10, wherein the bandwidths of the reference wavelength bands are different.

12. An apparatus as claimed in any one of claims 7 to 11 for the measuring the calorific content of alkane gases, wherein a single sample wavelength band and two reference wavelength bands are used, the sample wavelength band having a centre wavelength of $2300nm \pm 25nm$ (preferably $\pm 10nm$ and more preferably $\pm 5nm$) and a FWHM of $50nm \pm 20nm$ (preferably $\pm 10nm$ and more preferably $\pm 5nm$) and the reference wavelength bands having centre wavelengths of $2165nm \pm 25nm$ (preferably $\pm 10nm$ and more preferably $\pm 5nm$) and $2425nm \pm 25nm$ (preferably $\pm 10nm$ and more preferably $\pm 5nm$) and FWHMs of $25nm \pm 15nm$ (preferably $\pm 10nm$ and more preferably $\pm 5nm$) and the actual FWHM of the other reference band times ($1.2 \pm 0.1$) respectively.

13. A method of selecting filters for transmitting sample and reference wavelength bands for use in a two channel non-dispersive infrared detector for the measurement of the calorific value of a group of flammable gases ("target gases") in a gaseous space in circumstances in which the composition of the gas in the space may he unknown, the method comprising:

    a) selecting a sample filter transmitting at least one sample wavelength band, each of the target gases absorbing radiation in the at least one sample wavelength band;
    b) calculating the relationship between the intensity of radiation transmitted through the gas in the one or more sample wavelength hand and the calorific value for each target gas as compared to the said relationship of the other target gases, said relationship varying as between the target gases;
    c) selecting a reference filter transmitting one or more wavelength band, the target gases absorbing radiation in the at least one reference wavelength band but to a lesser extent than in the one or more sample wavelength band, said one or more reference wavelength band being chosen such that the intensity of radiation transmitted by each target gas in the one or more reference wavelength band is in inverse proportion to the relationship calculated in step b), whereby an approximate measure of the calorific value of the gas mixture irrespective of the exact composition of the gas in the gaseous space can be derived from the ratio of the intensity of the gas in the space in the at least one sample wavelength band to the intensity of the gas in the gas space in the at least one reference wavelength band;

    wherein the number of sample and reference wavelength bands is at least three.

# Fig.1.

# Fig.2.

## Response Curves

# Fig.3.

## Possible Response Homogenising Filter

# Fig.4.

## Response Curves

# Fig.5.